# EUROPÄISCHE PATENTSCHRIFT

(11) **EP 1 393 076 B1**
(45) Veröffentlichungstag und Bekanntmachung des Hinweises auf die Patenterteilung: **15.06.2005**
(21) Anmeldenummer: 02738068.2
(22) Anmeldetag: 13.05.2002
(51) Int. Cl.: G01N 33/564, G01N 33/566, G01N 33/68

(54) **VERFAHREN ZUR VORHERSAGE EINES TRANSPLANTATABSTOSSUNGSRISIKOS UND IMMUNOLOGISCHER TESTKIT**
METHOD FOR PREDICTING THE RISK OF TRANSPLANT REJECTION AND IMMUNOLOGICAL TESTKIT
PROCEDE DE PREVISION D'UN RISQUE DE REJET DE TRANSPLANTATION ET ENSEMBLE DE TEST IMMUNOLOGIQUE

(30) Priorität: 11.05.2001 DE 10123929
(43) Veröffentlichungstag der Anmeldung: 03.03.2004
(73) Patentinhaber: Celltrend GmbH, 14943 Luckenwalde (DE)
(72) Erfinder: SCHULZE-FORSTER, Kai, 14513 Teltow (DE); HEIDECKE, Harald, 14163 Berlin (DE)
(74) Vertreter: Ziebig, Marlene, Dr. Dipl.-Chem.
(86) Internationale Anmeldenummer: PCT/EP2002/005251
(87) Internationale Veröffentlichungsnummer: WO 2002/093171

(56) Entgegenhaltungen:
- WO-A-00/37075
- WO-A-00/39154
- DATABASE CA [Online] CHEMICAL ABSTRACTS SERVICE, COLUMBUS, OHIO, US; PITOTTI, A. ET AL: "HPLC method for evaluation of urinary angiotensin-converting enzyme: some examples of normal subjects and patients with renal transplantation" retrieved from STN Database accession no. 106:63376 CA XP002230925 & JOURNAL OF PHARMACEUTICAL AND BIOMEDICAL ANALYSIS (1986), 4(5), 677-83 , 1986,
- DATABASE MEDLINE [Online] Februar 1984 (1984-02) COLONNA J O 2ND ET AL: "Non-renin dependent hypertension in renal allograft rejections. A structural and functional analysis." Database accession no. NLM6365026 XP002230926 & ARCHIVES OF PATHOLOGY & LABORATORY MEDICINE. UNITED STATES FEB 1984, Bd. 108, Nr. 2, Februar 1984 (1984-02), Seiten 117-120, ISSN: 0003-9985
- OLDFIELD B J ET AL: "Efferent neural projections of angiotensin receptor (AT1) expressing neurones in the hypothalamic paraventricular nucleus of the rat." JOURNAL OF NEUROENDOCRINOLOGY. ENGLAND FEB 2001, Bd. 13, Nr. 2, Februar 2001 (2001-02), Seiten 139-146, XP002230924 ISSN: 0953-8194

## Beschreibung

Die Erfindung betrifft ein Verfahren und einen immunologischen Testkit zur Vorhersage eines Transplantatabstoßungsrisikos, indem anti-AT₁-Rezeptor-Autoantikörper in biologischen Materialien des zu untersuchenden Patienten nachgewiesen werden, beispielsweise durch eine Immunreaktion mit dem AT₁-Rezeptor oder funktionsanalogen Peptiden oder Proteinen desselben. Gegenstand der Erfindung ist auch die Verwendung des AT₁-Rezeptors oder funktionsanaloger Peptide oder Proteine zur Vorhersage (Diagnose) eines Transplantatabstoßungsrisikos. Dieses besteht dann, wenn anti-AT₁-Rezeptor-Autoantikörper in den biologischen Materialien, z.B. in Körperflüssigkeiten oder Geweben, nachweisbar sind.

Für eine erfolgreiche Organstransplantation ist es notwendig, dass das Spenderorgan histologisch möglichst weitgehend mit dem Empfängergewebe übereinstimmt. Diese Übereinstimmung wird durch das HLA-System (Abk. für (engl.) *h**uman* *l**eucocyte* *a**ntigen*) bestimmt. Dabei handelt es sich um ein komplexes System von Gewebeantigenen, die auf fast allen Zellen vorkommen. Dieses System spielt eine wichtige physiologische Rolle bei immunologischen Abwehrreaktionen (Erkennung von "Selbst" und "Nichtselbst"). Vor jeder Transplantation wird deshalb eine sogenannte Gewebetypisierung bei Organspender und -empfänger vorgenommen, um eine möglichst weitgehende HLA-Kompatibilität zu gewährleisten.

Aufgrund eines extremen genetischen Polymorphismus existiert eine außerordentlich große Anzahl verschiedener HLA-Moleküle. Eine vollständige Übereinstimmung wird ausschließlich bei eineiigen Zwillingen beobachtet, ansonsten sind HLA-Moleküle für jeden Menschen einzigartig.

Problematisch ist jedoch, dass trotz einer weitgehenden HLA-Übereinstimmung zwischen Empfänger und Spender eine Abstoßungsreaktion gegen das transplantierte Organ nicht ausgeschlossen werden kann.

A. Pitotti et al. (1986) offenbaren im Journal of Pharmaceutical and Biomedical Analysis 4 (5), 677-683, dass Angiotensin-umwandelnde Enzyme (ACE, EC 3.4.15.1) im Urin von Patienten nachweisbar sind, die verschiedene Krankheiten bzw. die sich einem Transplantateingriff unterzogen haben, wobei einige Patienten erhöhte aber die Mehrzahl der Patienten normale Werte an ACE aufweisen.

Die WO 00/39154 beschreibt Peptide des AT₁-Rezeptors und ihre Verwendung bei Präeklampsie und maligner Hypertonie. Die Peptide werden als Bestandteil des Kits beschrieben, der zur Diagnose der genannten Krankheiten eingesetzt werden kann.

Aufgabe der Erfindung ist es daher, ein effizientes und zuverlässiges Verfahren bereitzustellen, welches eine sichere und schnelle Vorhersagbarkeit des Risikos einer Transplantatabstoßungsreaktion ermöglicht.

Die vorliegende Erfindung löst dieses technische Problem durch die Bereitstellung eines solchen Verfahrens, bei dem anti-AT₁-Rezeptor-Autoantikörper aus biologischen Materialien des zu diagnostizierenden Patienten nachgewiesen werden und bei Nachweis der Autoantikörper ein Transplantatabstoßungsrisiko besteht bzw. vorhersagbar ist. Erfindungsgemäß bevorzugt erfolgt der Nachweis der Autoantikörper durch eine Immunreaktion mit dem AT₁-Rezeptor oder funktionsanalogen Peptiden oder Proteinen. Es ist aber auch möglich, die Autoantikörper über andere, dem Fachmann an sich bekannte, Verfahren nachzuweisen, z.B. über elektrophoretische Trennverfahren. Die Erfindung beruht auf dem überraschenden Nachweis, dass Patienten mit einer Transplantatabstoßungsreaktion ohne vorhersehbares immunologisches Risiko anti-AT₁-Rezeptor-Autoantikörper aufweisen.

Es konnte nachgewiesen werden, dass zwischen dem Vorhandensein der Autoantikörper und der Transplantatabstoßung ein Zusammenhang besteht. Anhand von zellbiologischen und immunhistochemischen Untersuchungen sowie der Untersuchung von Biopsiematerial konnte festgestellt werden, dass keine weiteren immunologischen Risikofaktoren für die Abstoßung des Transplantates bestanden. Es konnte aber gezeigt werden, dass das Vorhandensein der Autoantikörper zu Abstoßungsreaktionen bei transplantierten Organen im Empfänger führt.

Im Zusammenhang mit der vorliegenden Erfindung werden eine Reihe allgemeiner Begriffe wie folgt verwendet:
Ein "Transplantat" im Sinne der Erfindung ist ein transplantiertes oder zu transplantierendes Organ oder Gewebe. Im Sinne der Erfindung können Transplantate jedoch auch bestimmte Implantate sein, die aus Stoffen bzw. Teilen bestehen, die zur Erfüllung bestimmter Ersatzfunktionen für einen begrenzten Zeitraum oder auf Lebenszeit in einen Körper eingebracht werden. Die Implantate können beispielsweise aus anorganischer Materie bestehen, die mit organischen Substanzen, wie beispielsweise Knorpel oder Knochenzellen, beschichtet ist.
Unter einer "Transplantatabstoßung" gemäß der Erfindung ist die Induktion einer Immunreaktion des Empfängers auf das Transplantat zu verstehen. Eine Immunreaktion des Empfängers ist eine spezifische Schutz- oder Abwehrreaktion des Körpers gegen die Antigene des Transplantates.
Der "AT₁-Rezeptor" gemäß der Erfindung kann in seiner natürlichen zellulären Umgebung vorkommen und sowohl mit dem Material, mit dem er im natürlichen Zustand assoziiert ist, eingesetzt werden als auch isoliert eingesetzt werden. Der AT₁-Rezeptor ist dem Fachmann von seiner Primär-, Sekundär- und Tertiärstruktur bekannt. Bezogen auf das Gewicht des Gesamtrezeptors in der erfindungsgemäß einzusetzenden Präparation sollte der isolierte Rezeptor mindestens 0,5%, vorzugsweise mindestens 5%, besonders bevorzugt mindestens 25% und ganz besonders bevorzugt mindestens 50% ausmachen. Es ist bevorzugt, dass der Rezeptor isoliert eingesetzt wird, d. h. im Wesentlichen frei von anderen Proteinen, Lipiden, Kohlenhydraten oder anderen Substanzen, mit denen er natürlicher Weise assoziiert ist. Im Wesentlichen frei bedeutet, dass der Rezeptor zu mindestens 75%, vorzugsweise zu mindestens 85%, besonders bevorzugt zu mindestens 95% und ganz besonders bevorzugt zu mindestens 99% frei von anderen Proteinen, Lipiden, Kohlenhydraten oder anderen Substanzen ist, mit denen er natürlicher Weise assoziiert ist.

Im Zusammenhang mit der vorliegenden Erfindung können sowohl der natürlich vorkommende Rezeptor als auch alle Modifikationen, Mutanten oder Derivate des AT₁-Rezeptors eingesetzt werden. Auch der mittels Rekombinationstechniken hergestellte AT₁-Rezeptor, der Aminosäuremodifikationen, wie Inversionen, Deletionen, Insertion, Anlagerungen usw. enthält, kann zur erfindungsgemäßen Anwendung kommen, sofern mindestens ein Teil der essentiellen Funktion des AT₁-Rezeptors vorhanden ist, nämlich die Fähigkeit Antikörper zu binden. Der eingesetzte AT₁-Rezeptor kann auch ungewöhnliche Aminosäuren und/oder Modifikationen, wie eine Alkylierung, Oxidation, Thiolmodifikation, Denaturierung und Oligomerisation und dergleichen umfassen. Er kann auch chemisch synthetisiert werden. Erfindungsgemäß kann der AT₁-Rezeptor insbesondere ein Protein und/oder ein Peptid sein oder auch ein Fusionsprotein, das neben anderen Proteinen, Peptiden oder Teilen davon den AT₁-Rezeptor insgesamt oder teilweise enthält. Der Fachmann kann mittels üblicher Verfahren Peptide oder Polypeptide des AT₁-Rezeptor bestimmen, die funktionsanaloge Eigenschaften aufweisen. Beispielsweise besitzen diese Polypeptide oder Peptide 50%, 60%, 70% oder 80%, vorzugsweise 90%, stärker bevorzugt 95% und am meisten bevorzugt 98% Homologie zu den Peptiden, die als AT₁-Rezeptor identifiziert sind, wobei diese Homologie beispielsweise durch den "Smith-Waterman"-Homolgiesuche-Algorithmus, beispielsweise mit dem "MPSRCH"-Program (Oxford Molecular), bestimmt werden kann. Der in der vorliegenden Erfindung verwendete Begriff Peptid eines AT₁-Rezeptors umfasst Moleküle, die sich gegenüber der usprünglichen Sequenz durch Deletion(en), Insertion(en), Austausch(e) und/oder andere im Stand der Technik bekannte Modifikationen unterscheiden bzw. ein Fragment des ursprünglichen Aminosäuremoleküls umfassen, wobei der AT₁-Rezeptor noch die vorstehend erwähnten Eigenschaften aufweist. Dazu zählen auch Allelvarianten und Modifikationen. Verfahren zur Erzeugung der vorstehend Änderungen in der Aminosäuresequenz sind dem Fachmann bekannt und in Standardwerken der Molekularbiologie beschrieben, beispielsweise in Sambrook et al., supra. Der Fachmann ist auch in der Lage zu bestimmen, ob ein so veränderter AT₁-Rezeptor noch über die vorstehend erwähnten Eigenschaften verfügt. Mögliche Peptide des AT₁-Rezeptors, die erfindungsgemäß zum Einsatz kommen, können z.B. sein: AVHYQSN, SHFYQTR oder GYYFDTN. Alle vorstehend geschilderten Abwandlungen des AT₁-Rezeptors werden in der Beschreibung kurz mit dem Begriff "funktionsanaloge Peptide oder Proteine" bezeichnet.

"Biologische Materialien" im Sinne der Erfindung können alle biologischen Gewebe und Flüssigkeiten wie beispielsweise Blut, Lymphe, Urin, Gehirnflüssigkeit sein. Das biologische Material wird dem Patienten entnommen und der erfindungsgemäßen Diagnose unterzogen. Selbstverständlich ist es möglich, die Probe mit speziellen biochemischen und chemischen Mitteln bzw. Methoden zu behandeln oder für die Analyse vorzubereiten.

Die nachzuweisenden "Autoantikörper" im Sinne der Erfindung binden den AT₁-Rezeptor spezifisch. Die Autoantikörper können auch modifizierte Antikörper sein (z.B. oligomere, reduzierte, oxidierte und markierte Antikörper). Der in der vorliegenden Beschreibung verwendete Begriff Autoantikörper umfasst sowohl intakte Moleküle als auch Autoantikörper-Fragmente, wie Fab, F(ab')₂ und Fv, die bestimmte Epitop-Determinanten des AT₁-Rezeptors binden können. Bei diesen Fragmenten ist die Fähigkeit des Autoantikörpers zur selektiven Bindung seines Antigens oder Rezeptors teilweise erhalten geblieben, wobei die Fragmente wie folgt definiert sind:
(1) Fab, das Fragment, dass ein monovalentes Antigenbindungsfragment eines Antikörper-Moleküls enthält, lässt sich mittels Spaltung eines ganzen Antikörpers mit dem Enzym Papain erzeugen, wobei eine intakte leichte Kette und ein Teil einer schweren Kette erhalten werden;
(2) das Fab'-Fragment eines Antikörper-Moleküls lässt sich mittels Behandlung eines ganzen Antikörpers mit Pepsin und anschließender Reduktion gewinnen, wobei eine intakte leichte Kette und ein Teil der schweren Kette erhalten werden; pro Antikörper-Molekül werden zwei Fab'-Fragmente erhalten;
(3) F(ab')₂, das Fragment des Antikörpers, das sich mittels Behandlung eines ganzen Antikörpers mit dem Enzym Pepsin ohne anschließende Reduktion erhalten lässt; F(ab')₂ ist eine Dimer von zwei Fab'-Fragmenten, die durch zwei Disulfid-Bindungen zusammengehalten werden;
(4) Fv, definiert als gentechnisch verändertes Fragment, das den variablen Bereich der leichten Kette und den variablen Bereich der schweren Kette enthält und in Form von zwei Ketten exprimiert wird; und
(5) Einzelketten-Antikörper ("SCA"), definiert als gentechnisch verändertes Molekül, das den variablen Bereich der leichten Kette und den variablen Bereich der schweren Kette enthält, die durch einen geeigneten Polypeptid-Linker zu einem genetisch fusionierten Einzelketten-Molekül verbunden sind.

Der in der vorliegenden Erfindung verwendete Begriff Epitop bedeutet eine beliebige Antigen-Determinante auf dem AT₁-Rezeptor. Epitop-Determinanten bestehen normalerweise aus chemisch aktiven Oberflächen-Gruppierungen von Molekülen, wie Aminosäuren oder Zucker-Seitenketten, und besitzen normalerweise sowohl spezifische Merkmale der dreidimensionalen Struktur als auch spezifische Ladungsmerkmale.

Der Autoantikörper "bindet spezifisch" an den AT₁-Rezeptor oder zeigt damit eine spezifische Immunreaktivität, wenn der Autoantikörper in Gegenwart einer heterogenen Population von AT₁-Rezeptoren oder dessen Fragmenten in einer Bindungsreaktion seine Funktion ausübt, anhand der sich entscheiden lässt, ob der AT₁-Rezeptor oder eine andere biologische Struktur vorliegt. Unter den festgelegten Bedingungen eines Immuntests binden die angegebenen Autoantikörper vorzugsweise an einen speziellen Teil des AT₁-Rezeptors, während keine signifikante Bindung an andere in der Probe vorhandene Proteine erfolgt.

Unter "Patienten" werden im Sinne der Erfindung alle Menschen, Tiere, Pflanzen bzw. Mikroorganismen verstanden, unabhängig davon, ob sie pathologische Veränderungen aufweisen oder nicht. Sämtliche biologische Materialien, die aus Zellen, Geweben, Organen oder Organismen oder ähnlichem entnommen werden, können im Sinne der Erfindung biologisches Material des zu diagnostizierenden Patienten sein.

Eine "Immunreaktion" im Sinne der Erfindung ist eine spezifische Interaktion zwischen dem AT₁-Rezeptor oder funktionsanalogen Peptiden oder Proteinen und Autoantikörpern. Die Immunreaktion kann durch verschiedene Immunoassays nachgewiesen werden.

"Immunoassays" im Sinne der Erfindung sind Tests, die sich die spezifische Wechselwirkung zwischen dem AT₁-Rezeptor und funktionsanalogen Peptiden oder Proteinen und den Autoantikörpern zunutze machen, um die Gegenwart oder die Konzentration der Autoantikörper zu bestimmen. Die Detektion und Quantifizierung der Autoantikörper kann beispielsweise mit Hilfe der funktionsanalogen Peptide oder Proteine durchgeführt werden, wie z.B. durch eine Immunpräzipitation oder dem Immunblotting. Immunoassays im Sinne der Erfindung können z.B. in folgende Schritte unterteilt werden:
1) die Autoantikörper-AT₁-Rezeptor-Reaktion,
2) evtl. die Abtrennung des Autoantikörper-AT₁-Rezeptor-Komplexes von anderen Komponenten der Reaktionsmischung, insbesondere von ungebundenen Autoantikörpern und AT₁-Rezeptor, und
3) die Messung der Antwort.

Für die Autoantikörper-AT₁-Rezeptor-Reaktion sind verschiedene Konfigurationen möglich, beispielsweise
a) die Präzipitation eines Reaktanten mit einem Überschuss des anderen, oder
b) die Konkurrenz zwischen bekannten Mengen des Autoantikörpers oder AT₁-Rezeptors und dem Material, das untersucht werden soll.

Ein Test auf die Autoantikörper kann beispielsweise durchgeführt werden, durch
a) die Verwendung von AT₁-Rezeptoren/funktionsanalogen Peptiden oder Proteinen im Überschuss oder
b) die Konkurrenz eines markierten Autoantikörpers bekannter Menge und eines unmarkierten Antikörpers unbekannter Menge um eine bestimmte Menge an AT₁-Rezeptor oder funktionsanalogen Peptiden oder Proteinen.

Um die Abtrennung des Autoantikörper-AT₁-Rezeptor-Romplexes zu ermöglichen, kann der AT₁-Rezeptor an einen festen Träger immobilisiert sein. Das feste Trägermaterial kann beispielsweise Nitrozellulose, Polyvinylchlorid oder Polystyrol sein, so z.B. die Mulde einer Mikrotiterplatte. Zur Messung der Autoantikörper-AT₁-Rezeptor-Wechselwirkung können beispielsweise markierte Autoantikörper, markierte AT₁-Rezeptoren oder Sekundärreagenzien eingesetzt werden. Es ist beispielsweise möglich, den AT₁-Rezeptor radioaktiv bzw. mit Enzymen oder mit fluoreszierenden Verbindungen zu markieren. Unabhängig von der eingesetzten Markierung kann die Antwort der Autoantikörper-AT₁-Rezeptor-Wechselwirkung verstärkt werden, indem man sich die Affinität der Proteine Avidin oder Streptavidin zu Biotin zu nutze macht. Die erfindungsgemäß eingesetzten Immunoassays können sein:
1) Immuno-Assays unter Verwendung einer radioaktiven Markierung:
   a) Radioimmunoassays mit kompetiver Bindung (RIA) und
   b) Immunoradiometrischer Assay (IRMA),
2) Immunoassays unter Verwendung einer enzymatische Markierung:
   a) Enzymimmunoassays (EIA) und
   b) enzymgebundener Immunosorbentassay (ELISA);
3) Immunoassays unter Verwendung einer Kombination von Radioisotopen- und Enzymmarkierungen (ultrasensitiver Enzymradio-Immunoassay (USERIA)).

In einer Ausführungsform der Erfindung ist vorgesehen, dass als Autoantikörper humane IgA und/oder IgG-Autoantikörper nachgewiesen werden. Autoantikörper sind Glykoproteine, die auch als Immunglobuline bezeichnet werden. Die Antikörper des Menschen lassen sich in fünf Immunglobulinklassen unterscheiden. Immunglobuline der Klasse A (IgA) existieren sowohl in einer im Blut gelösten Form als auch in einer sekretorischen Variante. IgA besitzt zwei Grundklassen. Sekretorisches IgA besteht aus zwei Immunglobulingrundmolekülen zusammen mit einer J-Kette und einer sekretorischen Komponente. IgA-Moleküle können insbesondere in den Sekreten des Körpers vorherrschen. Die Immunglobuline der Klasse IgG stellen den größten Anteil der Immunglobuline. Die Antikörper der sekundären Immunantwort, die auf einen Kontakt des Immunsystems mit einem bestimmten Antigen erfolgt, gehören weitestgehend zu der Klasse IgG.

Um eine quantitative Aussage über die AT₁-Rezeptor-Autoantikörperreaktion machen zu können, muss einer der Reaktionspartner mit einer nachweisbaren Markierungssubstanz so gekoppelt werden, dass die immunologischen Eigenschaften der Komponenten weitgehend erhalten bleiben, beispielsweise durch das Streptavidin/Biotin-System oder unter Verwendung sekundärer Antikörper.

In einer weiteren bevorzugten Ausführungsform der Erfindung kann als Immunoassay eine Immunpräzipitation, ein Radioimmunoassay, ein enzymatischer Assay, ein Fluoreszenzimmunoassay, ein Chemilumineszenzimmunoassay, ein kompetitiver Bindungsassay, ein ELISA und/oder ein homogener Immunoassay eingesetzt werden, vorzugsweise ein ELISA. Bei der Immunpräzipitation kann die Reaktion zwischen dem Autoantikörper und dem AT₁-Rezeptor in vitro direkt am Gleichgewichtspunkt, bei dem die Konzentration der beiden Komponenten übereinstimmt, beobachtet werden. An diesem Punkt findet eine Immunpräzipitation statt. Die Immunpräzipitation kann beispielsweise in halbfesten Medien wie einem Agargel durchgeführt werden. Die Diffusion der immunchemischen Komponenten durch ein solches Gel erzeugt ein Konzentrationsgradienten, der sicherstellt, dass an einem bestimmten Punkt die Bedingung für eine Immunpräzipitation erfüllt sind, vorausgesetzt, dass sich Immunkomplexe bilden. Die Position und Beschaffenheit der Immunpräzipitate im Gel gibt Aufschluss über die Konzentration der Immunkomponenten. Einen qualitativen Hinweis auf eine Immunkomplexreaktion kann beispielsweise durch eine Immundoppeldiffusion erhalten werden. In ihrer einfachsten Form der Immunpräzipitation wird ein dünnes Agarosegel mit zwei kleinen Vertiefungen versehen, in die geringe Mengen des AT₁-Rezeptors und des biologischen Materials, das die Autoantikörper enthält, hineingegeben werden. Nach 24-stündiger Diffusion kann die Bildung der Immunkomplexe anhand einer weißen Präzipitatslinie zwischen den beiden Vertiefungen nachgewiesen werden. Eine aufwendigere Version stellt der Ochterlony-Test dar, bei dem auf der Gelplatte die Vertiefungen kreisförmig angeordnet sind, mit einer weiteren Vertiefung in der Kreismitte. Verschiedene Verdünnungen des biologischen Materials, das die Autoantikörper beinhaltet, werden in die äußeren Vertiefungen, die Lösung mit dem AT₁-Rezeptor/funktionsanalogen Peptiden in die Kreismitte gegeben.

Eine quantitative Analyse erlaubt die einfache Immundiffusion. Während eine Komponente homogen im Gel verteilt ist, kann die andere hierbei aus einer Vertiefung heraus diffundieren. Es ist beispielsweise möglich, die Lösung mit dem AT₁-Rezeptor/funktionsanalogen Peptiden mit in das Gel zu vermischen und in die Vertiefung eine Probe des biologischen Materials zu geben, bei dem untersucht werden soll, ob es den Autoantikörper beinhaltet, in dem sich ein Präzipitatring bildet.

Ein weiterer möglicher Assay zur Bestimmung der Autoantikörper ist der Radioimmunoassay (RIA). Der RIA ist ein empfindlicher Immunoassay, der auf der kompetitiven Bindung eines Antigens an einen Antikörper beruht, wobei der gebundene Anteil mit Hilfe von radioaktiv markierten Antigenen quantitativ bestimmt wird. Vorteilhafterweise können mit Hilfe des RIA Substanzmengen bis zum Pikogrammbereich bestimmt werden.

Ein weiterer Assay zur Bestimmung der Autoantikörper ist der enzymatische Immunoassay. Die Markierung eines Reaktionspartners, z.B. des Sekundärantikörpers, mit Enzymen bildet die Grundlage für diesen Immunoassay. Vorteilhaft sind Festphasensysteme wie Antikörper- oder Antigen-beschichtete Gefäße oder Kügelchen, da ihre Abtrennung nicht zur Denaturierung von Proteinen führt. Die Quantifizierung der gebundenen oder freien Phase erfolgt durch Messung der enzymatischen Aktivität. Es ist beispielsweise möglich, ein farbloses Substrat zuzufügen, das bei der Umwandlung durch ein Enzym ein farbiges Produkt ergibt, das spektroskopisch erfasst und mittels einer Standardprobe quantifiziert werden kann. Die Substratlösung wird in der Regel im Anschluß an die Trennung von gebundener und freier Phase zugegeben und nach einer vorgegebenen Zeit die Reaktion gestoppt und die optische Dichte des Produktes bestimmt. Dies ergibt ein Maß für die vorhandene Menge an Autoantikörper in der Probe. Es ist möglich, die Empfindlichkeit der Enzymreaktion auf verschiedenen Wegen zu erhöhen. Eine Methode ist das Prinzip der enzymatischen Verstärkung, bei der das Produkt der ersten enzymatischen Reaktion einen in sich geschlossenen Kreislauf in einem zweiten Enzymsystem erzeugt, was zu einer erhöhten Menge des farbigen Endproduktes führt. Ein Beispiel eines in sich geschlossenen Enzymkreislaufes ist das Enzym alkalische Phosphatase, das NADP⁺ zu NAD⁺ hydrolysiert, welches anschließend in einen Enzymkreislauf eintritt, um große Mengen eines farbigen Produktes von Nitrotetrazolium zu bilden. Weitere Enzyme können die Peroxidase oder die β-Galaktosidase sein. Als Substrat werden beispielsweise Luminol, β-Galaktose und p-Nitrophenylphosphat eingesetzt. Der Nachweis des gebundenen Autoantikörpers kann beispielsweise in einem zweiten Inkubationsschritt mit einem markierten anti-Autoantikörper erfolgen. Bei einem homogenen Immunoassay werden die gebildeten Immunokomplexe nicht abgetrennt.

Eine weitere Möglichkeit zum Nachweis der Autoantikörper besteht in der Verwendung eines Fluoreszenzimmunoassays, wobei fluoreszierende Marker die Basis des Assays bilden. Es ist möglich, die Bildung der Immunkomplexe beispielsweise anhand der Markierung von AT₁-Rezeptoren/funktionsanaloger Peptide der gebundenen oder freien Fraktion der immunchemischen Reaktionsmischung zu quantifizieren. Als Fluoreszenzträger sind beispielsweise Fluorescein oder Rhodamin einzusetzen bzw. Markierungen auf Basis von Chelatkomplexen seltener Erden, wie die organometallischen Koordinationskomplexe von Europium.

Die Autoantikörper können weiterhin in einem Chemilumineszenzimmunoassay detektiert werden, der vorteilhafterweise ein einfaches und stabiles Testsystem ist. Chemilumineszenz wird beobachtet, wenn eine hochenergetische chemische Reaktion Moleküle produziert, die sich in einem elektronisch angeregten Zustand befinden. Fallen diese angeregten Moleküle in ihren Grundstatus zurück, emittieren sie dabei Photonen. Derartige chemilumineszente Moleküle können beispielsweise an AT₁-Rezeptoren oder funktionsanaloge Peptide gekoppelt und im Immunoassay eingesetzt werden. Beispielsweise können die AT₁-Rezeptoren/funktionsanalogen Peptide mit Luminol oder Acridiniumsalzen markiert werden. Acridiniumsalze vollziehen Chemilumineszenzreaktionen, ohne dass ein Katalysator wie bei den Luminolderivaten notwendig ist. Die Katalysatoren umfassen eine große Bandbreit von Substanzen und von einfachen Übergangsmetallkationen bis hin zu komplexen Enzymen. Acridiniumsalze benötigen vorteilhalfterweise nur die Zugabe einer verdünnten alkalischen Wasserstoffperoxidlösung, um eine Chemilumineszenzreaktion auszulösen.

Es ist auch möglich, die Autoantikörper in einem kompetitiven Bindungstest zu detektieren, bei dem begrenzte Mengen des markierten AT₁-Rezeptors oder funktionsanalogen Peptides, die in Kompetition zu dem Autoantikörper stehen, eingesetzt werden. Eine solche Methode kann dann vorteilhaft sein, wenn ein gereinigter AT₁-Rezeptor oder funktionsanaloges Peptid für die Markierung zur Verfügung steht. Es kann beispielsweise ein AT₁-Rezeptor, gebunden an eine feste Phase, vorgelegt werden, an den der Autoantikörper, der in der Probe vorhanden ist, binden kann.

Die Erfindung betrifft auch die Verwendung des anti-AT₁-Rezeptors oder funktionsanaloger Peptide oder Proteine zur Vorhersage eines Transplantatabstoßungsrisikos. Hierzu werden die Autoantikörper in an sich bekannter Weise in den oben beschriebenen Immunoassays nachgewiesen.

Die Erfindung betrifft auch die Verwendung eines immunologischen Testkits, der den AT₁-Rezeptor oder funktionsanaloge Peptide oder Proteine desselben aufweist. Der erfindungsgemäße Testkit umfasst mindestens einen vollständigen AT₁-Rezeptor oder funktionsanaloge Peptide oder Proteine dieses Rezeptors, die gegebenenfalls an eine feste Phase gebunden sind. Weiterhin kann der Testkit auch Puffer, ein spezifisches Konjugat nebst Enzym, eine Waschlösung, eine Substratlösung zum Nachweis der Immunreaktion und/oder eine Stopplösung umfassen. Mit Hilfe derartiger Substanzen ist es dem Fachmann möglich, beispielsweise einen ELISA zur Detektion der Autoantikörper durchzuführen. Die Puffer, das spezifische Konjugat nebst Enzym, die Waschlösung, die Substratslösung zum Nachweis der Enzymreaktion und die Stopplösung sind dem Fachmann bekannt. Es wäre beispielsweise ausreichend, dass der Testkit einen gefriergetrockneten AT₁-Rezeptor oder AT₁-funktionsanaloge Peptide oder Proteine umfasst und erst kurz vor der Testung des biologischen Materials die Puffer und anderen Lösungen hinzugegeben werden. Es ist jedoch auch möglich, den AT₁-Rezeptor oder seine funktionsanalogen Peptide oder Proteine an eine feste Phase gebunden im Testkit vorzugeben. Für den Nachweis der Autoantikörper müssten dann spezifische Konjugate, Waschlösung, Substratlösung und Stopplösung, die Bestandteile des Testkits sein können, nach einem dem Fachmann bekannten Modus zugegeben werden.

Die Erfindung betrifft auch einen Testkit in Form eines Teststreifens, der den AT₁-Rezeptor oder funktionsanaloge Peptide oder Proteine desselben an einer festen Phase immobilisiert umfasst. Der Teststreifen kann beispielsweise in Serum - oder andere biologische Proben - eingetaucht und inkubiert werden. Anhand einer spezifischen biochemischen Reaktion auf dem Teststreifen nach Bildung des AT₁-Rezeptor-Autoantikörperkomplexes kann dann eine spezifische Farbreaktion ausgelöst werden, mit deren Hilfe die Autoantikörper detektiert werden können.

Das erfindungsgemäß Testsystem erlaubt es, die anti-AT₁-Rezeptor-Autoantikörper direkt im biologischen Material, z.B. im Patientenplasma, zu quantifizieren. Das erfindungsgemäße Nachweisverfahren ist zeitsparend und kostengünstig. Es ist die Bestimmung großer Probenmengen und durch den geringen apparativen Aufwand auch eine Durchführung in Routinelabors möglich.

Im folgenden soll die Erfindung anhand eines Ausführungsbeispieles veranschaulicht werden, ohne die Erfindung darauf einzuschränken:

### Beispiel AT1-ELISA:

Eine geeignete Streptavidin-beschichtete Mikrotiterplatte wird mit dem biotinylierten Peptid SAFHYESQNSTL beladen. Dazu werden 100 µL einer Lösung pro Vertiefung auf der Mikrotiterplatte mit 5 µg/mL in geeignetem Verdünnungspuffer inkubiert. Für die Messung der unspezifischen Bindung werden Vertiefungen parallel nur mit 100 µL Verdünnungspuffer gefüllt.

Nach der Reaktionszeit wird die Peptidlösung durch Ausgießen entfernt und die Vertiefungen werden dreimal mit ca. je 250 µl geeignetem Waschpuffer gewaschen.

Anschließend werden je 100 µl/Vertiefung der in Verdünnungspuffer verdünnten Seren sowohl auf die Peptidbeladene als auch die Vergleichsplatte gegeben und inkubiert. Die Vertiefungen werden danach wie oben beschrieben gewaschen.

Die gebundenen Antikörper werden mit einem Ziege-anti-human-Immunglobulin G-Antikörper nachgewiesen, an den Peroxidase gekoppelt ist. Dazu wird der Antikörper in Verdünnungspuffer verdünnt und inkubiert (100 µl/Vertiefung), woran sich drei Waschschritte anschließen (siehe oben).

Nach Zugabe von 100 µl einer gebrauchsfertigen Substratlösung (z. B. 3,3',5,5'-Tetramethylbenzidin) erfolgt eine Farbentwicklung in Abhängigkeit von der Peroxidasemenge in der Vertiefung. Die Substratreaktion wird durch Zugabe von 100 µl 0,5 M Schwefelsäure beendet. Die Absorption wird bei 450 nm gemessen.

Zur Auswertung wird die Differenz der Absorptionen von Peptid-beladener Mikrotiterplatte und der Vergleichsplatte ohne Peptid gebildet. Proben, deren Absorption größer ist als der cut-off, sind positiv. Der cut-off wird berechnet aus dem Mittelwert der Absorption von Negativspendern zuzüglich der dreifachen Standardabweichung. Im Test wird üblicherweise eine cut-off-Kontrolle oder eine Standardverdünnungsreihe mitgeführt, die eine Quantifizierung in relativen Einheiten ermöglicht.

## Patentansprüche

1. Verfahren zur Vorhersage eines Transplantatabstoßungsrisikos,
**dadurch gekennzeichnet, dass**
anti-AT₁-Rezeptor-Autoantikörper *in vitro* in biologischen Materialen des zu diagnostizierenden Patienten nachgewiesen werden, wobei beim Nachweis von Autoantikörpern ein Transplantatabstoßungsrisiko besteht.

2. Verfahren nach Anspruch 1,
**dadurch gekennzeichnet, dass**
der Nachweis durch eine Immunreaktion mit dem AT₁-Rezeptor oder funktionsanalogen Peptiden oder Proteinen vorgenommen wird.

3. Verfahren nach Anspruch 1 oder 2,
**dadurch gekennzeichnet, dass**
humane IgA- und/oder IgG-Autoantikörper nachgewiesen werden.

4. Verfahren nach einem der Ansprüche 1 bis 3, **dadurch**
**gekennzeichnet, dass**
der Nachweis der Autoantikörper in einem Immunoassay durchgeführt wird.

5. Verfahren nach Anspruch 4,
**dadurch gekennzeichnet, dass**
als Immunoassay eine Immunpräzipitation, ein Radioimmunoassay, ein enzymatischer Immunoassay, ein Fluoreszenzimmunoassay, ein Chemilumineszenzimmunoassay, ein kompetitiver Bindungsassay, ein ELISA oder ein homogener Immunoassay eingesetzt wird, vorzugsweise ein ELISA.

6. Verwendung des AT₁-Rezeptors oder funktionsanaloger Peptide oder Proteine zur in vitro Vorhersage eines Transplantatabstoßungsrisikos.

7. Verwendung nach Anspruch 6,
**dadurch gekennzeichnet, dass**
ein immunologischer Testkit, umfassend einen AT₁-Rezeptor oder funktionsanaloge Peptide oder Proteine desselben zur Vorhersage eines Transplantatabstoßungsrisikos eingesetzt wird.

8. Verwendung nach einem der Ansprüche 6 oder 7,
**dadurch gekennzeichnet, dass**
ein Teststreifen umfassend einen immobilisierten AT₁-Rezeptor oder funktionsanaloge Peptide oder Proteine desselben zur Vorhersage eines Transplantatabstoßungsrisikos eingesetzt wird.

9. Testkit zur Vorhersage eines Transplantatabstoßungsrisikos
**dadurch gekennzeichnet, dass**
er einen Teststreifen, auf welchem der AT₁-Rezeptor oder die funktionsanalogen Peptide oder Proteine desselben immobilisiert sind, umfasst.

## Claims

1. A method of predicting the risk of transplant rejection,
**characterized in that**
anti-AT₁ receptor auto-antibodies are detected in vitro in biological materials of a patient to be diagnosed, a risk of transplant rejection being present if auto-antibodies are detected.

2. The method according to claim 1,
**characterized in that**
detection is effected by means of an immune reaction using the AT₁ receptor or functionally analogous peptides or proteins.

3. The method according to claim 1 or 2,
**characterized in that**
human IgA and/or IgG auto-antibodies are detected.

4. The method according to any of claims 1 to 3,
**characterized in that**
detection of the auto-antibodies is effected in an immunoassay.

5. The method according to claim 4,
**characterized in that**
an immunoprecipitation, a radioimmunoassay, an enzyme immunoassay, a fluorescent immunoassay, a chemiluminescent immunoassay, a competitive binding assay, an ELISA, or a homogeneous immunoassay is used as immunoassay, preferably an ELISA.

6. Use of the AT₁ receptor or functionally analogous peptides or proteins to predict the risk of transplant rejection in vitro.

7. The use according to claim 6,
**characterized in that**
a immunological test kit comprising a AT₁ receptor or functionally analogous peptides or proteins thereof for predicting the risk of transplant rejections is used.

8. The use according to claim 6 or 7,
**characterized in that**
a test strip having AT₁ receptor or functionally analogous peptides or proteins thereof immobilized thereon for predicting the risk of transplant rejection is used.

9. A test kit for predicting the risk of transplant rejection,
**characterized in that**
the test kit comprises a test strip having the AT₁ receptor or functionally analogous peptides or proteins thereof immobilized thereon.

## Revendications

1. Procédé de prédiction d'un risque de rejet d'un greffon,
**caractérisé en ce**
**qu'**on détecte des autoanticorps anti-récepteurs AT₁ *in vitro* dans une matière biologique du patient à diagnostiquer, moyennant quoi si l'on détecte les autoanticorps, il existe un risque de rejet du greffon.

2. Procédé selon la revendication 1,
**caractérisé en ce que**
la détection est réalisée par une réaction immune avec le récepteur AT₁ ou des peptides ou des protéines ayant une fonction analogue.

3. Procédé selon la revendication 1 ou 2,
**caractérisé en ce**
**qu'**on détecte des autoanticorps IgA et/ou IgG humains.

4. Procédé selon une des revendications 1 à 3,
**caractérisé en ce que**
la détection des autoanticorps s'effectue par un dosage immunologique.

5. Procédé selon la revendication 4,
**caractérisé en ce**
**qu'**en tant que dosage immunologique, on effectue une immunoprécipitation, un dosage radio-immunologique, un dosage immunoenzymatique, un dosage immunofluorescent, un dosage immunologique par chimioluminescence, un dosage par compétition de liaison, un dosage ELISA ou un dosage immunologique homogène, de préférence un dosage ELISA.

6. Utilisation du récepteur AT₁ ou des peptides ou protéines ayant une fonction analogue pour prédire *in vitro* un risque de rejet d'un greffon.

7. Utilisation selon la revendication 6,
**caractérisée en ce**
**qu'**on utilise un kit d'essai immunologique comprenant un récepteur AT₁ ou des peptides ou protéines ayant une fonction analogue de celui-ci pour prédire un risque de rejet de greffon.

8. Utilisation selon une des revendications 6 ou 7,
**caractérisée en ce**
**qu'**on utilise une bandelette d'essai comprenant un récepteur AT₁ immobilisé ou des peptides ou protéines ayant une fonction analogue de celui-ci pour prédire un risque de rejet de greffon.

9. Kit d'essai destiné à prédire un risque de rejet de greffon,
**caractérisé en ce**
**qu'**il comprend une bandelette d'essai, sur laquelle le récepteur AT₁ ou les peptides ou les protéines ayant une fonction analogue à celui-ci sont immobilisés.
